(19) Europäisches Patentamt
European Patent Office
Office européen des brevets

(11) **EP 3 635 133 B1**

(12) **EUROPEAN PATENT SPECIFICATION**

(45) Date of publication and mention of the grant of the patent:
**05.08.2026 Bulletin 2026/32**

(21) Application number: **18814347.3**

(22) Date of filing: **11.06.2018**

(51) International Patent Classification (IPC):
**G16B 20/00** $^{(2019.01)}$    **G06F 7/00** $^{(2006.01)}$
**G16B 30/00** $^{(2019.01)}$    **G16B 40/00** $^{(2019.01)}$

(52) Cooperative Patent Classification (CPC):
(C-Sets available)
**G16B 40/00; G06F 7/00; G16B 20/00; G16B 30/00;**
C12Q 1/6869; C12Q 1/6886; G16B 35/00;
G16B 50/10                                  (Cont.)

(86) International application number:
**PCT/US2018/036950**

(87) International publication number:
**WO 2018/227202 (13.12.2018 Gazette 2018/50)**

(54) **DETERMINATION OF CANCER TYPE IN A SUBJECT BY PROBABILISTIC MODELING OF CIRCULATING NUCLEIC ACID FRAGMENT ENDPOINTS**

BESTIMMUNG DES KREBSTYPS BEI EINER PERSON DURCH PROBABILISTISCHE MODELLIERUNG VON ZIRKULIERENDEN NUKLEINSÄUREFRAGMENT-ENDPUNKTEN

DÉTERMINATION DU TYPE DE CANCER CHEZ UN SUJET PAR MODÉLISATION PROBABILISTE DE POINTS D'EXTRÉMITÉ DE FRAGMENT D'ACIDE NUCLÉIQUE CIRCULANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2017 US 201762517820 P**

(43) Date of publication of application:
**15.04.2020 Bulletin 2020/16**

(73) Proprietor: **Bellwether Bio, Inc.**
**Seattle, WA 98104 (US)**

(72) Inventors:
• **SNYDER, Matthew, William**
**Seattle**
**WA 98104 (US)**

• **DEAN, Jason, Thaddeus**
**Seattle**
**WA 98104 (US)**

(74) Representative: **Carpmaels & Ransford LLP**
**One Southampton Row**
**London WC1B 5HA (GB)**

(56) References cited:
**WO-A2-2016/015058       WO-A2-2016/015058**
**US-A1- 2015 368 708     US-A1- 2016 019 338**
**US-A1- 2016 371 431     US-A1- 2017 024 513**
**US-B2- 6 925 389**

(52) Cooperative Patent Classification (CPC): (Cont.)

C-Sets
C12Q 1/6869, C12Q 2535/122, C12Q 2537/165

**Description**

FIELD

Provided are methods for diagnosis of cancer using cell-free DNA.

BACKGROUND

Cell-free DNA (cfDNA) is present in the circulating plasma, urine, and other bodily fluids of humans, cfDNA contains both single and double stranded DNA fragments that are relatively short and are normally found at low concentrations in plasma. In the circulating plasma of healthy individuals, cfDNA is believed to derive from apoptosis of blood cells. However, other tissues can contribute to cfDNA in plasma.

In recent years, efforts have been made to exploit cfDNA in conjunction with the emergence of new technologies related to cost-effective DNA sequencing in the development of diagnostics. In pregnant women, for example, a proportion of cfDNA in circulating plasma derives from fetal or placental cells. Screening for genetic abnormalities in the fetus, such as chromosomal trisomies, can be achieved by deep sequencing of the cfDNA of a pregnant woman, since the ciDN A of a pregnant woman is a mixture of cfDNA derived from the maternal and fetal genomes. One can expect to observe an excess of reads mapping to chromosome 21 if the fetus has trisomy 21. Non-invasive screening based on analysis of cfDNA is now routinely offered to pregnant women.

With respect to cancer diagnostics, a proportion of cfDNA in circulating plasma can come from a tumor, with the contribution from the tumor often increasing with cancer stage. Cancer is caused by abnormal cells exhibiting uncontrolled proliferation secondary to mutations in their genomes. The observation of mutations in cfDNA has substantial promise to effectively serve as a diagnostic for cancer.

With respect to transplant rejection, after a transplant is performed, there is a risk of allograft rejection. Currently, the gold standard for assessing transplant rejection involves an invasive biopsy. A major challenge is determining whether and to what extent a rejection is occurring without an invasive biopsy. Recently, using cfDNA from the donor as a non-invasive marker for detecting allograft rejection has been explored.

There are several shared characteristics of current cfDNA diagnostic efforts. First, each relies on sequencing of cfDNA, generally from circulating plasma but potentially from other bodily fluids. Second, each relies on the fact that cfDNA comes from cell populations bearing genomes that differ very little from one another with respect to primary nucleotide sequence and/or copy number. Third, the basis for each is to detect or monitor genotypic differences between cell populations.

The reliance of cfDNA efforts in diagnostics on what are essentially genotypic differences is the basis of their success but also a major limitation. For example, since an overwhelming majority of cfDNA corresponds to regions of the human genome that are identical, the reliance on genotypic differences is uninformative when one is trying to discriminate between cell populations or between one group of subjects and another. WO 2016/015058 (University of Washington) discusses *"methods of determining tissues and/or cell types giving rise to cell-free DNA, and methods of identifying a disease or disorder using same"* (title),

There, is a need for a cfDNA test with greater discriminatory power.

SUMMARY

Provided herein are cfDNA based methods for determining the type of cancer as set out in the appended set of claims.

In particular, the invention provides a method for determining type of cancer in a subject in need thereof, the method comprising:

a. isolating cfDiNA from biological sample(s) from one or more subjects with a first cancer, the isolated cfDNA comprising a first plurality of cfDNA fragments:
b. constructing a first sequencing library from the first plurality of cfDNA fragments;
c. sequencing first fragment endpoints of the first plurality of cfDNA fragments:
d. determining genomic locations of the first fragment endpoints within a reference genome for at least some of the first plurality of cfDNA fragments as a function of the sequences;
e. determining at least one first training sample for the first fragment endpoints, wherein the at least one first training sample comprises a first vector corresponding to the number of first fragment endpoints observed at each respective genomic location;
f. isolating cfDNA from biological sample(s) from one or more subjects with a second cancer, the cfDNA comprising a second plurality of cfDNA fragments;

g. constructing a second sequencing library from the second plurality of cfDNA fragments;

h. sequencing second fragment endpoints of the second plurality of cfDNA fragments;

i. determining genomic locations of the second fragment endpoints within the reference genome for at least some of the second plurality of cfDNA fragments as a function of the sequences;

j. determining at least one second training sample for the second fragment endpoints, wherein the at least one second training sample comprises a second vector corresponding to the number of second fragment endpoints observed at each respective genomic location;

k. isolating cfDNA from a biological sample from the subject, the isolated cfDNA comprising a sample plurality of cfDNA fragments;

l. constructing a sample sequencing library from the sample plurality of cfDNA fragments;

m. sequencing sample fragment endpoints of the sample plurality of cfDNA fragments;

n. determining genomic locations of the sample fragment endpoints within the reference genome for at least some of the sample plurality of cfDNA fragments as a function of the sequences;

o. assigning to each of the sample fragment endpoints a sample vector corresponding to the number of sample cfDNA fragment endpoints observed at the genomic location;

p. calculating at least one first probability score for the sample vector and the first vector and at least one second probability score for the sample vector and the second vector, each calculated according to a multinomial probability formula: and

q. determining type of cancer in the subject as

i. the first cancer if the at least one first probability score is higher than that at least one second probability score: or
ii. the second cancer if the at least one second probability score is higher that at least one first probability score.

In some embodiments, the probability scores are calculated according to a multinomial formula in linear space. In some embodiments, the probability scores are calculated according to a multinomial probability formula in logarithmic space. In some embodiments, a label is added to the match the determined cancer type.

In some embodiments, at least some of the cfDNA fragments are subjected to a size selection to retain only cfDNA fragments having a length between an upper bound and a lower bound. In some embodiments, the upper bound is 200, 190, 180, 170, 160, 150, 140, 130, 120, 110, 100, 90, 80, 70, 60, or 50 base pairs and the lower bound is 20, 25, 30, 35, 36, 40, 45, 50, 60, 70, 80, 90. 100, 110, or 120 base pairs.

In some embodiments, a subset of isolated cfDNA fragments from the subject is targeted to a genomic location. In some embodiments, genomic location comprises one or more genomic annotations. In some embodiments, the one or more genomic annotations comprises or consists of transcription start sites (TSSs).

In some embodiments, the method further comprises generating a report listing a plurality of probability scores calculated for the biological sample from the subject using either or both of the at least one first training sample and/or the at least one second training sample. In some embodiments, the method any of the above claims further comprises recommending treatment for the identified disease or condition in the subject.

In some embodiments, the biological sample comprises or consists of whole blood, peripheral blood plasma, urine, or cerebral spinal fluid.

BRIEF DESCRIPTION OF THE FIGURES

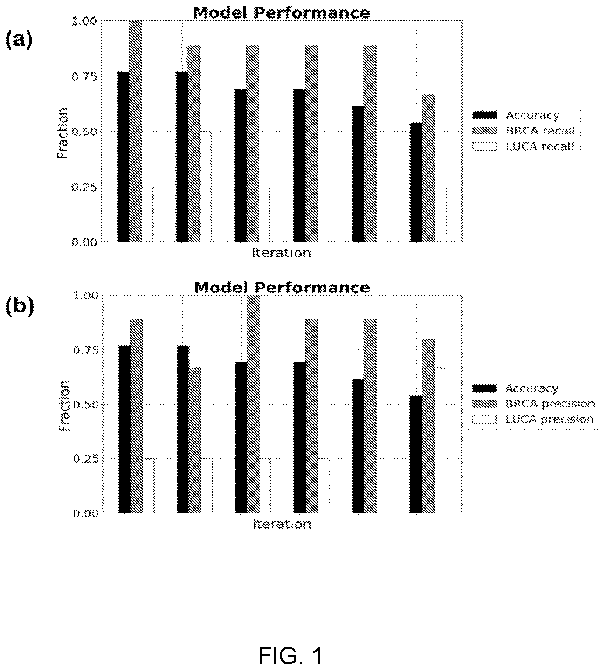

FIG. 1 depicts the results from testing a total of 49 samples, 18 samples from each of two cancer types were randomly selected for training, with the remaining 13 samples being held out for testing.

DESCRIPTION OF EXEMPLARY EMBODIMENTS

The present invention provides methods for determining the type of cancer in a subject already diagnosed with cancer and for determining both whether a subject has or does not have cancer. If the subject has cancer, the present method determines the type of cancer.

I. Definitions

As used herein, "allotransplantation" refers to the transplantation of cells, tissues, or organs, to a recipient from a genetically non-identical donor of the same species. The transplant is called an allograft, allogeneic transplant, or homograft. Most human tissue and organ transplants are allografts.

As used herein. "annotations" "DNA annotations," "genome annotation," "or "genomic annotations" refer to the locations of genes, coding regions, and functional areas and the determination of what those genes, coding regions, and functional areas do.

As used herein, "autoimmune disease" refers to a condition resulting from an abnormal immune response to a normal body part.

As used herein, "burden" refers to a load or weight with respect to a particular disease or physiological state. In particular, a burden is normally used to indicate an increased load or weight of a disease or physiological condition.

As used herein, "cancer" refers to disease caused by an uncontrolled division of abnormal cells in a part of the body.

As used herein, "cell-free DNA" or "cfDNA" refers to DNA fragments present in the blood plasma.

As used herein, "fragment endpoints" or "endpoints" shall refer to the termini of cfDNA.

As used herein, "genome" or "genomic" refers to the complete set of genes or genetic material present in a cell or organism.

As used herein, "inflammatory bowel disease" refers to group of chronic intestinal diseases characterized by inflammation of the bowel in the large or small intestine. The most common types of inflammatory bowel disease are ulcerative colitis and Crohn's disease.

As used herein, "myocardial infarction" refers to the irreversible death or necrosis of heart muscle secondary to prolonged lack of oxygen supply.

As used herein, "next generation sequencing" refers to any high-throughput sequencing approach including, but not limited to, one or more of the following: massivelyparallel signature sequencing, pyrosequencing (e.g., using a Roche 454 sequencing device), Illumina sequencing, sequencing by synthesis, ion torrent sequencing, sequencing by ligation ("SOLiD"), single molecule real-time ("SMRT") sequencing, colony sequencing, DNA nanoball sequencing, heliscope single molecule sequencing, and nanopore sequencing.

As used herein, "peripheral blood" refers to the flowing, circulating blood of the body. It is normally composed of erythrocytes, leukocytes, and thrombocytes. These blood cells are suspended in blood plasma, through which the blood cells are circulated through the body. Peripheral blood is different from the blood whose circulation is enclosed within the liver, spleen, bone marrow, and the lymphatic system. These areas contain their own specialized blood.

As used herein, "peripheral blood plasma" refers to the plasma found in peripheral blood.

As used herein, "plasma" or "blood plasma" refers to the liquid component of blood that normally holds the blood cells in whole blood in suspension. Holding blood cells in whole blood makes plasma the extracellular matrix of blood cells.

As used herein, "stroke" refers to the sudden death of brain cells due to lack of oxygen, caused by blockage of blood flow or rupture of an artery to the brain.

As used herein, "vector" shall refer to points arising from the number of fragment endpoints observed at each genomic location. In mathematics, a vector is conceived as an object that has both a magnitude and a direction. A vector as used herein, then, has a magnitude of the ntimber of fragment endpoints at a given location and a direction determined with respect to genomic location.

As used herein, "whole blood" refers to blood drawn directly from the body from which no components, such as plasma or platelets, have been removed.

## II. Subjects

A subject may be any subject known to one skilled in the art. In some embodiments, the subject is human. In some embodiments, the subject is non-human. A human subject can be any gender, such as male or female. In some embodiments, the human can be an infant, child, teenager, adult, or elderly person. In some embodiments, the subject is a female subject who is pregnant, suspected of being pregnant, or planning to become pregnant.

In some embodiments, the subject is a mammal, a non-human mammal, a non-human primate, a primate, a domesticated animal (e.g., laboratory animals, household pets, or livestock), or a non-domesticated animals (e.g., wildlife). In some embodiments, the subject is a dog, cat, rodent, mouse, hamster, cow, bird, chicken, pig, horse, goat, sheep, rabbit, ape, monkey, or chimpanzee.

## III. Biological Samples

Biological samples can be any type known to one skilled in the art and may be obtained from any subject. In some embodiments, the biological sample is from a human subject. In some embodiments, the biological sample is from a non-human subject. In some embodiments, a biological sample is isolated from one or more subjects having one or more physiological states. In some embodiments, the one or more physiological states are one or more healthy human states and/or human disease states.

In some embodiments, biological samples comprise or consist of unprocessed samples (e.g., whole blood, tissue, or cells) or processed samples (e.g., serum or plasma). In some embodiments, biological samples are enriched for a certain type of nucleic acid. In some embodiments, biological samples are processed to isolate nucleic acids from other components within the biological sample.

In some embodiments, biological samples comprise cells, tissue, a bodily fluid, or a combination thereof. In some embodiments, biological samples comprise or consist of whole blood, peripheral blood plasma, urine, or cerebral spinal fluid. In some embodiments, biological samples comprise or consist of a blood components, plasma, serum, synovial fluid, bronchial-alveolar lavage, saliva, lymph, spinal fluid, nasal swab, respiratory secretions, stool, peptic fluids, vaginal fluid, semen, and/or menses.

In some embodiments, biological samples comprise or consist of fresh samples. In some embodiments, biological samples comprise or consist of frozen samples. In some embodiments, biological samples comprise fixed samples, e.g., samples fixed with a chemical fixative such as formalin-fixed paraffin-embedded tissue.

Biological samples may also be obtained at any point during medical care. In some embodiments, biological samples are obtained prior to treatment, during the treatment process, after diagnosis, or any other point. Biological samples may be obtained at specific intervals, such as daily, weekly, or monthly, or during a routine medical examination.

### IV. Isolating cfDNA

Isolation of cfDNA can proceed according any method known to those of skill in the art. For example, the QIAGEN QIAamp Circulating Nucleic Acid kit is commonly used to isolate cfDNA from plasma or urine based on binding of cfDNA to a silica column. Isolation may also include phenol-chloroform extraction followed by isopropanol or ethanol precipitation.

In some embodiments, isolating cfDNA is done in such a manner as to maximize the recovery of short fragments (<100 base pairs), as the composition of short fragments differs more strongly between healthy and disease states than the composition of longer fragments does between healthy and disease samples. In some embodiments, any of the cfDNA fragments are subjected to a size selection to retain only cfDNA fragments having a length between an upper bound and a lower bound. In some embodiments, the upper bound is 200, 190, 180, 170, 160, 150, 140, 130, 120, 110, 100, 90, 80, 70, 60, or 50 base pairs and the lower bound is 20, 25, 30, 35, 36, 40, 45, 50, 60, 70, 80, 90, 100, 110, or 120 base pairs. In some embodiments, only the lower bound is 36 and the upper bound is 100.

### V. Constructing a Sequencing Library

After isolating cfDNA from a biological sample, isolated cfDNA comprising a plurality of cfDNA fragments can be subjected to one or more enzymatic steps to create a sequencing library. Enzymatic steps can proceed according to techniques known to those of skill in the art. Enzymatic steps may include 5' phosphorylation, end repair with a polymerase, A-tailing with a polymerase, ligation of one or more sequencing adapters with a ligase, and linear or exponential amplification with a polymerase.

Preparation of sequencing libraries may be performed to maximize the conversion of short fragments (<100 base pairs). In some embodiments, a physical size-selection step is employed to select for short cfDNA fragments. In some embodiments, an enrichment step is employed, wherein the enrichment step comprises enriching cfDNA that are targeted to a genomic location. An enrichment step may be employed by itself or in conjunction with a physical size-selection step. A physical size selection step could comprise or consist of gel electrophoresis and/or capillary electrophoresis. In some embodiments, constructing a sequencing library should preserve the original termini of cfDNA fragments.

Some embodiments comprise attaching adapters to the plurality of cfDNA fragments to aid in purification, detection, amplification, or a combination thereof. In some embodiments, the adapters are sequencing adapters. In some embodiments, at least some of the plurality of cfDNA fragments are attached to the same adapter. In some embodiments, different adaptors are attached at both ends of the plurality of cfDNA fragments. In some embodiments, at least some of the plurality of cfDNA fragments may be attached to one or more adapters on one end. Adapters may be attached to cfDNAs by primer extension, reverse transcription, or hybridization.

In some embodiments, an adapter is attached to a plurality of cfDNA fragments by ligation. In some embodiments, an adapter is attached to a plurality of cfDNA fragments by a ligase. In some embodiments, an adapter is attached to a plurality of cfDNA fragments by sticky-end ligation or blunt-end ligation. An adapter may be attached to the 3' end, the 5' end, or both ends of the plurality of cfDNA fragments.

In some embodiments, enzymatic end-repair processes are used for adapter ligation. The end repair reaction may be performed by using one or more end repair enzymes (e.g., a polymerase and an exonuclease).

In some embodiments, the ends of the plurality of cfDNA fragments can be polished by treatment with a polymerase. Polishing can involve removal of 3' overhangs, fillin of 5' overhangs, or a combination thereof. For example, a polymerase may fill in the missing bases for a DNA strand from 5' to 3' direction. The polymerase can be a proofreading polymerase (e.g., comprising 3' to 5' exonuclease activity). The proofreading polymerase can be, e.g., a T4 DNA polymerase, Pol 1 Klenow fragment, or Pfu polymerase. Polishing can comprise removal of damaged nucleotides using any means known in the art. In some embodiments, the ends of the plurality of cfDNA fragments are polished by treatment with an exonuclease to remove the 3' overhangs.

## VI. Sequencing of Fragment Endpoints

In some embodiments, sequencing fragment endpoints of the plurality of cfDNA fragments comprises or consists of sequencing the plurality of cfDNA fragments. In some embodiments, sequencing fragment endpoints of the plurality of cfDNA fragments comprises or consists of sequencing an entire cfDNA fragment(s) of the plurality of cfDNA fragments. In some embodiments, sequencing fragment endpoints of the plurality of cfDNA fragments comprises or consists of sequencing only the fragment endpoints of the plurality of cfDNA fragments.

Following the preparation of a sequencing library, at least the fragment endpoints of the plurality of cfDNA fragments are sequenced. Any method known to one skilled in the art may be used to generate a dataset consisting of at least one "read" (the ordered list of nucleotides comprising each sequenced molecule). In some embodiments, sequencing fragment endpoints comprises or consists of next generation sequencing assay.

In some embodiments, sequencing comprises or consists of classic Sanger sequencing methods that are well known in the art. In some embodiments, sequencing comprises or consists of sequencing on an Illumina Novaseq instrument with an S4 flow cell. In some embodiments, sequencing comprises or consists of sequencing on Illumina's Genome Analyzer IIX, MiSeq personal sequencer. NextSeq series, or HiSeq systems, such as those using HiSeq 4000, HiSeq 3000. HiSeq 2500, HiSeq 1500, HiSeq 2000, or HiSeq 1000. In some embodiments, sequencing comprises or consists of using technology available by 454 Lifesciences, Inc. to sequence fragment endpoints. In some embodiments, sequencing comprises or consists of ion semiconductor sequencing (e.g., using technology from Life Technologies (Ion Torrent)).

In some embodiments, sequencing comprises or consists of nanopore sequencing (See e.g., Soni GV and Meller A (2007) Clin Chem 53: 1996-2001). In some embodiments, nanopore sequencing comprises or consists of using technology from Oxford Nanopore Technologies: e.g., a GridION system. In some embodiments, nanopore sequencing comprises or consists of strand sequencing in which intact DNA polyniers can be passed through a protein nanopore with sequencing in real time as the DNA translocates the pore.

In some embodiments, nanopore sequencing comprises or consists of exonuclease sequencing in which individual nucleotides can be cleaved from a DNA strand by a processive exonuclease and the nucleotides can be passed through a protein nanopore. In some embodiments, nanopore sequencing comprises or consists of nanopore sequencing technology from GENIA. In some embodiments nanopore sequencing comprises or consists of technology from NABsys. In some embodiments, nanopore sequencing comprises or consists of technology from IBM/Roche.

In some embodiments, sequencing comprises or consists of sequencing by ligation approach. One example is the next generation sequencing method of SOLiD sequencing. SOLiD may generate hundreds of millions to billions of small sequence reads at one time.

## VII. Determining a Genomic Location of Fragment Endpoints

For each dataset (i.e., for each sequenced library of a plurality of fragment endpoints), the two genomic endpoints of each sequenced fragment endpoints are extracted with computer software. After sequencing of cfDNA fragments and fragment endpoints and appropriate quality control, a genomic location for the fragment endpoints within a reference genome is determined. The process of determining genomic locations, or mapping, identifies the genomic origin of each fragment based on a sequence comparison, determining, for example, that a given fragment of cfDNA was originally part of a specific region of chromosome 12. Determining a genomic location of fragment endpoints can be done with any human reference genome, such as, for example, Genbank hg19 or Genbank hg38. using bwa software (See, http://bio-bwa. sourceforge.net/: See, WO 2016/015058).

The procedure is performed for each library derived from each biological sample to produce one dataset per library. The procedure of mapping provides two fragment endpoints for each cfDNA fragment. The fragment endpoints are given numerical values ("coordinates"), representing the specific offset, relative to one end of a chromosome, of the fragment endpoint's location within the reference genome.

In some embodiments, fragment endpoints are further oriented in two dimensions, such that for every fragment endpoint, a given fragment endpoint's coordinate is either greater than or less than its partner's coordinate. In other words, each fragment endpoint is the left-most or right-most fragment endpoint coordinate of the pair in two-dimensional space. In some embodiments, a plurality of the fragment endpoints are classified based on the strand, for example Watson or Crick, from which their associated, sequenced cfDNA fragment was derived

In some embodiments, the genomic location of the first fragment endpoints and the second reference fragment endpoints may be determined with an available database. In some embodiments, the available database comprises or consists of a public database.

The method according to the invention may be shortened when using an available database. When using an available database, some embodiments comprise a method for determining type of cancer in a subject in need thereof, comprising:

a. determining genomic locations of first fragment endpoints within a reference genome using available database fragment endpoints, the first fragment endpoints corresponding to at least one first cancer;

b. determining at least one first training sample for the first fragment endpoints, wherein the at least one first training sample comprises a first vector corresponding to the number of first fragment endpoints observed at each respective genomic location;

c. determining genomic locations of second fragment endpoints within a reference genome using available database fragment endpoints, the second fragment endpoints corresponding to at least one second cancer;

d. determining at least one second training sample for the second fragment endpoints, wherein the at least one second training sample comprises a second vector corresponding to the number of second fragment endpoints observed at each respective genomic location;

e. isolating cfDNA from a biological sample from the subject, the cfDNA comprising a sample plurality of cfDNA fragments;

f. constructing a sample sequencing library from the sample plurality of cfDNA fragments;

g. sequencing sample fragment endpoints of the sample plurality of cfDNA fragments;

h. determining genomic locations of the sample fragment endpoints within the reference genome for at least some of the sample plurality of cfDNA fragments as a function of the sequences;

i. assigning to the sample fragment endpoints a sample vector corresponding to the number of sample cfDNA fragment endpoints observed at the genomic location;

j. calculating at least one first probability score for the sample vector and the first vector and at least one second probability score for the sample vector and the second vector, each calculated according to a multinomial probability formula; and

k. determining the type of cancer in the subject as

    i. the first cancer if the at least one first probability score is higher than the at least one second probability score; or
    ii. the second cancer if the at least one second probability score is higher that at least one first probability score.

## VIII. Determining a Vector

Vectors are determined with the number of fragment endpoints observed at each genomic location. Some embodiments comprise a set of two or more vectors, each having a single entry for a single coordinate under consideration. In some embodiments, for example, the physiological states comprise healthy human state. In some embodiments, the physiological states comprise a human disease state.

Within each vector, integer counts at each coordinate are converted to relative frequencies by dividing each integer count value by the sum of all integer count values in a vector. For example, if the sum of all integer counts in a vector is 1000, and the first three coordinates in the vector have integer counts of 1, 4, and 0, the resulting relative frequencies will be 1/1000, 4/1000, and 0/1000, respectively. The process is repeated for each vector representing each physiological state. The resulting relative frequency values for the given set of coordinates and for a physiological state comprise a vector for the physiological state.

In some embodiments, the set of two or more vectors are visualized. In some embodiments, the set of two of more vectors are visualised as a two-dimensional histogram or scatterplot.

In some embodiments, vectors are normalized to correct for differences in sequencing depth or coverage, fragment length distribution, local GC content, and chromosome number between the first physiological state, the second physiological state, and the subject. Normalization can be performed using standard techniques known to those skilled in the art.

## IX. Selecting Fragment Endpoints and Genomic Annotations

In some embodiments, the method further comprises filtering isolated cfDNA to retain cfDNA having a length between an upper bound and a lower bound. In some embodiments, the upper bound is 200, 190, 180, 170, 160, 150, 140, 130, 120, 110, 100, 90, 80, 70, 60, or 50 base pairs and the lower bound is 20, 25, 30, 35, 36, 40, 45, 50, 60, 70, 80, 90, 100, 110, or 120 base pairs. In some embodiments, only fragments falling within a specified length range, such as 36-100 base pairs, are retained. In some embodiments, filtering comprises gel electrophoresis and/or capillary electrophoresis.

In some embodiments, a subset of isolated cfDNA is targeted to a genomic location. In some embodiments, the genomic location comprises one or more genomic annotations. In some embodiments, the one or more genomic annotations comprises DNA-binding or DNA-contacting proteins.

Genomic annotations enrich genomic locations by providing functional information related to location in the genome. Once a genome is sequenced it can be annotated to make sense of it. For DNA annotation, a previously unknown sequence of genetic material is enriched with information relating genomic position to intron-exon boundaries, regulatory sequences, repeats, gene names, and protein products. The National Center for Biomedical Ontology (www.bioontology.org) develops tools for annotation of database records based on the textual descriptions of those records.

In some embodiments, the one or more genomic annotations comprises or consists of transcription start sites. A transcription start site is the location where transcription starts at the 5'-end of a gene sequence. As the starting place for transcription, proteins involved in transcription may be expected to affect and influence fragment endpoints, especially between one physiological state and another.

In some embodiments, the one or more genomic annotations comprises or consists of nucleosomes. Nucleosomes are known to be positioned in relation to landmarks of gene regulation, for example transcriptional start sites and exonintron boundaries.

## X. Physiological States and Conditions

According to the invention, the disease or physiological condition, at least one first physiological state, or at least one second physiological state comprises or consists of cancer. In some embodiments, cancer comprises or consists of acute lymphoblastic leukemia; acute myeloid leukemia; acute myeloid leukemia; adrenocortical carcinoma; AIDS-Related cancers: anal cancer; astrocytomas; central nervous system cancers; basal cell carcinoma; bile duct cancer: bladder cancer; bone cancers: brain stem glioma; brain tumors; craniopharyngioma; ependymoblastoma; medulloblastoma: medulloepithelioma; pineal parenchymal tumors: neuroectodermal tumors: breast cancer; bronchial tumors; Burkett's lymphoma; gastrointestinal cancers: cervical cancers; chronic lymphocytic leukemia; chronic myelogenous leukemia; chronic myeloproliferative disorders; colon cancer; colorectal cancer, cutaneous T-Cell lymphomas: endometrial cancers: esophageal cancers: Ewing cancers: extracranial germ cell tumors; eye cancers: retinoblastoma: gallbladder cancers: gastric cancers; gastrointestinal stromal tumor (GIST): ovarian cancers; hairy cell leukemia; head and neck cancer; heart cancer, hepatocellular cancers: Hodgkin's lymphoma; Kaposi's sarcoma: kidney cancers: lip and oral cavity cancers: liver cancers: lung cancers; non-small cell lung cancer; lymphoma; Waldenström macroglobulinemia; melanomas; mesothelioma; metastatic squamous neck cancers: mouth cancers; nasopharyngeal cancers: neuroblastoma: ovarian cancers: pancreatic cancer; penile cancers; pituitary tumors; rectal cancers: salivary gland cancers: squamous cell carcinomas; stomach cancers; throat cancers; thyroid cancers; and vaginal cancers. In some embodiments, cancer consists of breast cancer or non-small cell lung cancer.

In some embodiments, the at least one first physiological state consists of a cancer at a first clinical stage (e.g., stage I) and the at least one second physiological state consists of a cancer at a second clinical stage (e.g., stage IV). In some embodiments, the first clinical stage consists of a cancer at stage 0, stage I, stage II, stage III, or stage IV. In some embodiments, the second clinical stage consists of a cancer at stage 0. stage I, stage II, stage III, or stage IV.

## XI. Calculating Probability Scores

In some embodiments, the at least one first probability score for the sample vector and the first vector and/or the at least one second probability score for the sample vector and the second vector is calculated according to a multinomial probability formula and the type of cancer in the subject is determined as the first cancer if the at least one first probability score is higher than that at least one second probability score or the second cancer if the at least one second probability score is higher that at least one first probability score. In some embodiments, the probability scores are calculated according to a multinomial formula in linear space. In some embodiments, the probability scores are calculated according to a multinomial probability formula in logarithmic space.

The procedure for assigning a label to the sample is based on calculating the probabilities of the observed number of fragment endpoints at each coordinate in the sample given the probabilities from the two or more training samples. In some embodiments, this calculation is similar to a classic "urn" problem in statistics, in which an urn is filled with red and blue marbles, each with a certain proportion, and the calculation finds the probability of specific number of red marbles being chosen when at least that number of marbles are randomly selected from the urn.

With respect to the current invention, if there are two coordinates (here denoted A and B) in each vector, and three fragment endpoints are sampled from these two coordinates, the possible distributions of these fragment endpoints are {A:0; B:3}; {A:1; B:2}; {A:2; B:1}; and {A:3: B:0}. In a sample, if the distribution of fragment endpoints is {A:1; B:2}, the probability can be calculated based on the allocation of fragment endpoints to each coordinate in the training sample, where fragment endpoints in the training sample distribution are analogous to colored marbles in the urn, and fragment endpoints in the sample are analogous to the randomly selected set of marbles from urn. In this example, there are multiple urns, each having a different proportion of red and blue marbles, such that the randomly sampled set of marbles is most likely to have been drawn from one specific urn.

In some embodiments, the at least one probability is calculated according to the following multinomial probability formula:

$$P(n_1, n_2, \ldots, n_k | N, c) = \binom{N}{n_1, n_2, \ldots, n_k} \prod_{i=1}^{k} p_{i,c}^{n_i} \qquad \text{(Equation 1)}$$

Here, $N$ refers to the total number of fragment endpoint observations at selected coordinates in the sample (e.g., if there are 50 genomic coordinates, and two observations at each coordinate, then $N$ is 2 x 50 = 100). $n_i$ refers to the number of fragment endpoint observations at coordinate $i$ in the sample. $k$ denotes the total number of coordinates in the vector. $c$ refers to the training sample distribution for a physiological state such that $p_{i,c}$ represents the probability in training sample distribution $c$ for fragment endpoint coordinates $i$. $p_{i,c}$ values are taken from the coordinate probability vector derived from training samples for the physiological state for which the sample probability is being calculated.

In some embodiments, to make the equation computationally tractable for large N in terms of both time and numerical precision, the one or more probabilities are calculated in logarithm-space, using the same notation as in the previous formula according to the formula:

$$\log(P(n_1, n_2, \ldots, n_k | N, c)) = \log(N!) - \sum_{i=1}^{k} \log(n_i!) + \sum_{i=1}^{k} n_i \log(p_{i,c}) \quad \text{(Equation 2)}$$

$p_{i,c}$ may have a value of 0 for one or more coordinates in the coordinate probability vector, thus making $\log(p_{i,c})$ undefined. In certain embodiments, when $p_{i,c}$ is 0, its value is changed to a small, positive, non-zero value to allow calculation of the probability.

After calculating the at least one first probability score for the sample vector and the first vector and the at least one second probability score for the sample vector and the second vector, the sample is assigned a label by selecting the largest probability value and labelling the sample with the physiological state from which the largest probability value was derived. For example, if there are two training samples, one derived from training samples from subjects with breast cancer and the other derived from subjects with lung cancer, and the calculated probabilities of a sample are 0.03 when using the breast cancer training sample and 0.02 when using the lung cancer training sample, the sample receives a label of breast cancer.

In some embodiments, a label is only applied to a sample when the maximum calculated probability meets or exceeds a certain threshold value. If the maximum probability falls below a threshold value, no label is applied.

A threshold value can be determined by one skilled in the art. In certain embodiments, a label is only applied if the percentage or absolute difference between a maximum calculated probability and a second-largest calculated probability exceeds a certain threshold. If the percentage or absolute difference falls below the threshold, no label is applied.

In some embodiments, many physiological conditions can be analysed simultaneously.

## XII. Computer Systems

Some embodiments comprise a computer system programmed to implement the methods provided herein. The computer system includes a central processing unit ("CPU"). The computer system also includes memory or memory location, electronic storage unit, communication interface for communicating with other systems, and peripheral devices, such as cache, other memory, data storage, and/or electronic display adapters. The memory, storage unit, interface, and peripheral devices are in communication with the CPU through a communication bus, such as a motherboard.

The storage unit can be a data storage unit. The computer system can be operatively coupled to a computer network. The network can be the Internet, an intranet and/or extranet, or an intranet and/or extranet that is in communication with the Internet. The network in some cases is a telecommunication and/or data network. The network can include one or more computer servers, which can enable distributed computing, such as cloud computing.

The CPU can execute a sequence of instructions, which can be embodied in a program or software. The instructions may be stored in the memory. The instructions can be directed to the CPU.

The computer system can include or be in communication with an electronic display that comprises a user interface for providing a report, which may include a diagnosis of a subject or a therapeutic intervention for the subject. The report may be provided to a subject, a health care professional, a lab-worker, or other individual.

## XIII. Diagnosis, Reports, and Treatment

Some embodiments comprise providing a report, and recommending treatment for the disease or physiological condition. An electronic report with scores can be generated to indicate diagnosis or prognosis. A diagnosis of a particular disease or physiological condition may then be made by a qualified healthcare practitioner. If an electronic report indicates there is a treatable disease, the electronic report can prescribe a therapeutic regimen or a treatment plan.

## EXAMPLES

Example 1

Frozen human plasma specimens were obtained in 3 x 1 ml aliquots from each of 49 donors with clinical diagnosis of breast cancer (n=27) or non-small cell lung cancer (n=22). The specimens were thawed on the benchtop to approximately room temperature. Each specimen was processed in one batch with the Qiagen Circulating Nucleic Acid kit as per the manufacturer's protocol. Briefly, each plasma sample was placed in a 50 ml conical and combined with 300 ul Proteinase K and 2.4 ml Buffer ACL (lysis buffer). The tubes were vortexed for 30 seconds, covered with parafilm, and placed in a 60°C water bath for 30 minutes. After incubation, the tubes were placed on the bench, and 5.4 mL of Buffer ACB (binding buffer) was added to each sample, followed by vortexing for 30 seconds. The tubes were then placed on ice for 10 minutes. The full volume of each tube was loaded into a spin column with tube extender in a Qiagen vacuum manifold. Each column was washed with 600 ul ACW1, 750 $\mu$l ACW2, and 750 ul 100% ethanol. The columns were spun at 17000 x g for 3 minutes and the flowthrough was discarded. The columns were dried at room temperature with the lids open for 10 minutes. 40 ul of buffer AVE (elution buffer) was added to each column and incubated at room temperature for 10 minutes to elute the DNA. The DNA was collected in Lo-Bind tubes (Eppendorf) by centrifugation at 17000 x g for 2 minutes. cfDNA yield was quantified by a Qubit fluorometer (Invitrogen) using a dsDNA HS kit. The purified cfDNA samples were then stored at -20°C.

To prepare sequencing libraries, a maximum of 30 ng of cfDNA in 10 $\mu$l buffer AVE was used as input. The indexed libraries were constructed using the ThruPLEX Plasma-seq kit (Rubicon Genomics) as per the manufacturer's protocol, comprising a proprietary series of end-repair, adapter ligation, and amplification steps. Library amplification was monitored with real-time PCR to avoid overamplification. After amplification, PCR products were cleaned with AMPure beads (Beckman Coulter) and eluted in 20 ul of buffer EB. Library fragment size was determined by gel electrophoresis, and library concentration was determined by Qubit using a dsDNA HS kit. Libraries were pooled and diluted for sequencing on an Illumina Novaseq instrument with an S4 flow cell.

Paired-end, 2 x 100 base pair reads were generated for the pooled libraries. After sequencing, the resulting sequencing data was split by sample index. Adapters were trimmed using the software *cutadapt*. The trimmed reads were aligned to the human reference genome (version hg38) with the software *bwa*.

Two genomic coordinates representing the fragment endpoints of each properly paired fragment having mapping quality of at least 60 were extracted using a custom software program. Only fragments having inferred lengths between 36 and 100 base pairs (inclusive) were considered.

From a total of 49 samples, 18 samples from each of the two cancer types were randomly selected for training samples, with the remaining 13 samples being held out as samples. The random selection was repeated six times, with the same number of training samples and samples selected in each iteration.

FIG. 1 shows the results of the testing the model on the held out samples for each iteration. The dark bar depicts accuracy; BRCA depicts breast cancer; LUCA depicts lung cancer. The y-axis depicts fraction.

**Claims**

1.    A method for determining type of cancer in a subject in need thereof, the method comprising:

> a. isolating cell-free DNA (cfDNA) from biological sample(s) from one or more subjects with a first cancer, the isolated cfDNA comprising a first plurality of cfDNA fragments;
> b. constructing a first sequencing library from the first plurality of cfDNA fragments;
> c. sequencing first fragment endpoints of the first plurality of cfDNA fragments;
> d. determining genomic locations of the first fragment endpoints within a reference genome for at least some of the first plurality of cfDNA fragments as a function of the sequences;
> e. determining at least one first training sample for the first fragment endpoints, wherein the at least one first training sample comprises a first vector corresponding to the number of first fragment endpoints observed at each respective genomic location;
> f. isolating cfDNA from biological sample(s) from one or more subjects with a second cancer, the cfDNA comprising a second plurality of cfDNA fragments;
> g. constructing a second sequencing library from the second plurality of cfDNA fragments;
> h. sequencing second fragment endpoints of the second plurality of cfDNA fragments;
> i. determining genomic locations of the second fragment endpoints within the reference genome for at least some of the second plurality of cfDNA fragments as a function of the sequences;
> j. determining at least one second training sample for the second fragment endpoints, wherein the at least one second training sample comprises a second vector corresponding to the number of second fragment endpoints observed at each respective genomic location;

k. isolating cfDNA from a biological sample from the subject, the isolated cfDNA comprising a sample plurality of cfDNA fragments;

l. constructing a sample sequencing library from the sample plurality of cfDNA fragments;

m. sequencing sample fragment endpoints of the sample plurality of cfDNA fragments;

n. determining genomic locations of the sample fragment endpoints within the reference genome for at least some of the sample plurality of cfDNA fragments as a function of the sequences;

o. assigning to each of the sample fragment endpoints a sample vector corresponding to the number of sample cfDNA fragment endpoints observed at the genomic location;

p. calculating at least one first probability score for the sample vector and the first vector and at least one second probability score for the sample vector and the second vector, each calculated according to a multinomial probability formula; and

q. determining type of cancer in the subject as

    i. the first cancer if the at least one first probability score is higher than that at least one second probability score; or

    ii. the second cancer if the at least one second probability score is higher that at least one first probability score.

2. The method of claim 1, further comprising the step of applying a label to match the determined cancer type.

3. The method of claim 1 or claim 2, wherein any of the cfDNA fragments are subjected to a size selection to retain only cfDNA fragments having a length between an upper bound and a lower bound.

4. The method of claim 3, wherein the upper bound is 200, 190, 180, 170, 160, 150, 140, 130, 120, 110, 100, 90, 80, 70, 60, or 50 base pairs and the lower bound is 20, 25, 30, 35, 36, 40, 45, 50, 60, 70, 80, 90, 100, 110, or 120 base pairs.

5. The method of any one of the preceding claims, wherein a subset of isolated cfDNA fragments from the subject are targeted to a genomic location.

6. The method of claim 5, wherein the genomic location comprises one or more genomic annotations.

7. The method of claim 6, wherein the one or more genomic annotations comprises or consists of transcription start sites (TSSs).

8. The method of any one of the preceding claims, further comprising providing a report, for example wherein the report lists a plurality of probability scores calculated for the sample using either or both of the at least one first training sample and/or the at least one second training sample.

9. The method any one of the preceding claims, further comprising recommending treatment for the identified disease or condition in the subject.

10. The method of any one of the preceding claims, wherein the biological sample comprises or consists of whole blood, peripheral blood plasma, urine, or cerebral spinal fluid.

**Patentansprüche**

1. Verfahren zur Bestimmung eines Krebstyps bei einem Individuum, das dies benötigt, wobei das Verfahren Folgendes umfasst:

a. Isolieren von zellfreier DNA (cfDNA) aus einer oder mehreren biologischen Proben von einem oder mehreren Individuen mit einer ersten Krebserkrankung, wobei die isolierte cfDNA mehrere erste cfDNA-Fragmente umfasst;

b. Konstruieren einer ersten Sequenzierungsbibliothek aus den mehreren ersten cfDNA-Fragmenten;

c. Sequenzieren erster Fragmentendpunkte der mehreren ersten cfDNA-Fragmente;

d. Bestimmen genomischer Orte der ersten Fragmentendpunkte innerhalb eines Referenzgenoms für mindestens einige der mehreren ersten cfDNA-Fragmente in Abhängigkeit von den Sequenzen;

e. Bestimmen mindestens einer ersten Training-Probe für die ersten Fragmentendpunkte, wobei die mindestens eine erste Training-Probe einen ersten Vektor umfasst, der der Anzahl an jedem betreffenden genomischen Ort beobachteter erster Fragmentendpunkte entspricht;

f. Isolieren von cfDNA aus einer oder mehreren biologischen Proben von einem oder mehreren Individuen mit einer zweiten Krebserkrankung, wobei die cfDNA mehrere zweite cfDNA-Fragmente umfasst;

g. Konstruieren einer zweiten Sequenzierungsbibliothek aus den mehreren zweiten cfDNA-Fragmenten;

h. Sequenzieren zweiter Fragmentendpunkte der mehreren zweiten cfDNA-Fragmente;

i. Bestimmen genomischer Orte der zweiten Fragmentendpunkte innerhalb des Referenzgenoms für mindestens einige der mehreren zweiten cfDNA-Fragmente in Abhängigkeit von den Sequenzen;

j. Bestimmen mindestens einer zweiten Training-Probe für die zweiten Fragmentendpunkte, wobei die mindestens eine zweite Training-Probe einen zweiten Vektor umfasst, der der Anzahl an jedem betreffenden genomischen Ort beobachteter zweiter Fragmentendpunkte entspricht;

k. Isolieren von cfDNA aus einer biologischen Probe von dem Individuum, wobei die isolierte cfDNA mehrere Probe-cfDNA-Fragmente umfasst;

l. Konstruieren einer Probe-Sequenzierungsbibliothek aus den mehreren Probe-cfDNA-Fragmenten;

m. Sequenzieren von Probe-Fragmentendpunkten der mehreren Probe-cfDNA-Fragmente;

n. Bestimmen genomischer Orte der Probe-Fragmentendpunkte innerhalb des Referenzgenoms für mindestens einige der mehreren Probe-cfDNA-Fragmente in Abhängigkeit von den Sequenzen;

o. Zuordnen eines der Anzahl an dem genomischen Ort beobachteter Probe-cfDNA-Fragmentendpunkte entsprechenden Probe-Vektors zu jedem der Probe-Fragmentendpunkte;

p. Berechnen mindestens einer ersten Wahrscheinlichkeitswertung für den Probe-Vektor und den ersten Vektor und mindestens einer zweiten Wahrscheinlichkeitswertung für den Probe-Vektor und den zweiten Vektor, wobei diese jeweils gemäß einer Wahrscheinlichkeits-Multinomialformel berechnet werden; und

q. Bestimmen des Krebstyps bei dem Individuum als

    i. die erste Krebserkrankung, falls die mindestens eine erste Wahrscheinlichkeitswertung höher als die mindestens eine zweite Wahrscheinlichkeitswertung ist; oder

    ii. die zweite Krebserkrankung, falls die mindestens eine zweite Wahrscheinlichkeitswertung höher als die mindestens eine erste Wahrscheinlichkeitswertung ist.

2. Verfahren nach Anspruch 1, ferner umfassend den Schritt, bei dem eine zu dem bestimmten Krebstyp passende Markierung angewandt wird.

3. Verfahren nach Anspruch 1 oder Anspruch 2, wobei die cfDNA-Fragmente jeweils einer Größenauswahl unterzogen werden, so dass nur cfDNA-Fragmente mit einer Länge zwischen einer Obergrenze und einer Untergrenze behalten werden.

4. Verfahren nach Anspruch 3, wobei die Obergrenze bei 200, 190, 180, 170, 160, 150, 140, 130, 120, 110, 100, 90, 80, 70, 60 oder 50 Basenpaaren und die Untergrenze bei 20, 25, 30, 35, 36, 40, 45, 50, 60, 70, 80, 90, 100, 110 oder 120 Basenpaaren liegt.

5. Verfahren nach einem der vorhergehenden Ansprüche, wobei eine Teilmenge isolierter cfDNA-Fragmente von dem Individuum auf einen genomischen Ort gerichtet ist.

6. Verfahren nach Anspruch 5, wobei der genomische Ort eine oder mehrere genomische Annotationen umfasst.

7. Verfahren nach Anspruch 6, wobei die eine oder mehreren genomischen Annotationen Transkriptionsstartstellen (TSS) umfassen oder daraus bestehen.

8. Verfahren nach einem der vorhergehenden Ansprüche, ferner umfassend Bereitstellen eines Berichts, wobei beispielsweise der Bericht mehrere Wahrscheinlichkeitswertungen auflistet, die für die Probe unter Verwendung der mindestens einen ersten Training-Probe oder/und der mindestens einen zweiten Training-Probe berechnet wurden.

9. Verfahren nach einem der vorhergehenden Ansprüche, ferner umfassend Empfehlen einer Behandlung gegen die identifizierte Krankheit bzw. das identifizierte Leiden bei dem Individuum.

10. Verfahren nach einem der vorhergehenden Ansprüche, wobei die biologische Probe Vollblut, peripheres Blutplasma, Urin oder Liquor umfasst oder daraus besteht.

**Revendications**

1. Procédé pour déterminer le type de cancer chez un sujet nécessitant cela, le procédé comprenant :

   a. isoler de l'ADN acellulaire (ADNc) à partir d'échantillon(s) biologique(s) d'un ou plusieurs sujets atteints d'un premier cancer, l'ADNc isolé comprenant une première pluralité de fragments d'ADNc ;
   b. construire une première banque de séquençage à partir de la première pluralité de fragments d'ADNc ;
   c. séquencer des premiers points d'extrémité de fragments de la première pluralité de fragments d'ADNc ;
   d. déterminer des emplacements génomiques des premiers points d'extrémité de fragments au sein d'un génome de référence pour au moins certains de la première pluralité de fragments d'ADNc en fonction des séquences ;
   e. déterminer au moins un premier échantillon d'apprentissage pour les premiers points d'extrémité de fragments, l'au moins un premier échantillon d'apprentissage comprenant un premier vecteur correspondant au nombre de premiers points d'extrémité de fragments observés au niveau de chaque emplacement génomique respectif ;
   f. isoler de l'ADNc à partir d'échantillon(s) biologique(s) provenant d'un ou plusieurs sujets atteints d'un second cancer, l'ADNc comprenant une seconde pluralité de fragments d'ADNc ;
   g. construire une deuxième banque de séquençage à partir de la deuxième pluralité de fragments d'ADNc ;
   h. séquencer des deuxièmes points d'extrémité de fragments de la deuxième pluralité de fragments d'ADNc ;
   i. déterminer des emplacements génomiques des deuxièmes points d'extrémité de fragments au sein du génome de référence pour au moins certains de la seconde pluralité de fragments d'ADNc en fonction des séquences ;
   j. déterminer au moins un deuxième échantillon d'apprentissage pour les deuxièmes points d'extrémité de fragments, l'au moins un deuxième échantillon d'apprentissage comprenant un deuxième vecteur correspondant au nombre de deuxièmes points d'extrémité de fragments observés au niveau de chaque emplacement génomique respectif ;
   k. isoler de l'ADNc à partir d'un échantillon biologique provenant du sujet, l'ADNc isolé comprenant une pluralité d'échantillons de fragments d'ADNc ;
   l. construire une banque de séquençage d'échantillons à partir de la pluralité d'échantillons de fragments d'ADNc ;
   m. séquencer des points d'extrémités de fragments d'échantillons de la pluralité d'échantillons de fragments d'ADNc ;
   n. déterminer des emplacements génomiques des points d'extrémité de fragments d'échantillons dans le génome de référence pour au moins certains de la pluralité d'échantillons de fragments d'ADNc en fonction des séquences ;
   o. attribuer à chacun des points d'extrémité de fragments d'échantillons un vecteur d'échantillon correspondant au nombre de points d'extrémités de fragments d'ADNc d'échantillons observés au niveau de l'emplacement génomique ;
   p. calculer au moins un premier score de probabilité pour le vecteur d'échantillons et le premier vecteur et au moins un deuxième score de probabilité pour le vecteur d'échantillons et le deuxième vecteur, chacun calculé selon une formule de probabilité multinomiale ; et
   q. déterminer le type de cancer chez le sujet comme étant

      i. le premier cancer si l'au moins un premier score de probabilité est supérieur à au moins un deuxième score de probabilité ; ou
      ii. le deuxième cancer si l'au moins un deuxième score de probabilité est supérieur à au moins un premier score de probabilité.

2. Procédé selon la revendication 1, comprenant en outre l'étape d'application d'un marqueur pour correspondre au type de cancer déterminé.

3. Procédé selon la revendication 1 ou la revendication 2, dans lequel de quelconques des fragments d'ADNc sont soumis à une sélection de taille pour ne retenir que des fragments d'ADNc ayant une longueur entre une limite supérieure et une limite inférieure.

4. Procédé selon la revendication 3, dans lequel la limite supérieure est 200, 190, 180, 170, 160, 150, 140, 130, 120, 110, 100, 90, 80, 70, 60, ou 50 paires de bases et la limite inférieure est 20, 25, 30, 35, 36, 40, 45, 50, 60, 70, 80, 90, 100, 110 ou 120 paires de bases.

**5.** Procédé selon l'une quelconque des revendications précédentes, dans lequel un sous-ensemble de fragments d'ADNc isolés provenant du sujet sont ciblés vers un emplacement génomique.

**6.** Procédé selon la revendication 5, dans lequel l'emplacement génomique comprend une ou plusieurs annotations génomiques.

**7.** Procédé selon la revendication 6, dans lequel l'annotation ou les annotations génomiques comprennent ou sont constituées de sites de début de transcription (TSS).

**8.** Procédé selon l'une quelconque des revendications précédentes, comprenant en outre la fourniture d'un rapport, par exemple dans lequel le rapport liste une pluralité de scores de probabilité calculés pour l'échantillon en utilisant l'un ou l'autre ou les deux parmi l'au moins un premier échantillon d'apprentissage et/ou l'au moins un second échantillon d'apprentissage.

**9.** Procédé selon l'une quelconque des revendications précédentes, comprenant en outre une recommandation de traitement pour la maladie ou l'affection identifiée chez le sujet.

**10.** Procédé selon l'une quelconque des revendications précédentes, dans lequel l'échantillon biologique comprend ou est constitué de sang entier, de plasma de sang périphérique, d'urine ou de liquide céphalorachidien.

FIG. 1

## REFERENCES CITED IN THE DESCRIPTION

**Patent documents cited in the description**

- WO 2016015058 A

**Non-patent literature cited in the description**

- **SONI GV** ; **MELLER A**. *Clin Chem*, 2007, vol. 53, 1996-2001